# EUROPÄISCHE PATENTSCHRIFT

(11) **EP 0 433 852 B1**
(45) Veröffentlichungstag und Bekanntmachung des Hinweises auf die Patenterteilung: **13.03.1996**
(21) Anmeldenummer: 90123827.9
(22) Anmeldetag: 11.12.1990
(51) Int. Cl.: A61B 17/58, A61B 17/80

(54) **Gitter für die Osteosynthese oder zum Befestigen von künstlichen Körperteilen**
Grid for osteosynthesis or for attaching of artificial members of the body
Grille pour osthéosynthèse ou pour la fixation d'éléments artificiels remplaçant des parties du corps

(30) Priorität: 22.12.1989 DE 3942676
(43) Veröffentlichungstag der Anmeldung: 26.06.1991
(73) Patentinhaber: Leibinger GmbH, D-79111 Freiburg (DE)
(72) Erfinder: Leibinger, Karl, W-7200 Tuttlingen-Möhringen (DE); Leibinger, Franz, W-7202 Mühlheim-Stetten (DE)
(74) Vertreter: von Hellfeld, Axel, Dr. Dipl.-Phys.

(56) Entgegenhaltungen:
- DE-U- 8 807 909
- FR-A- 742 618
- US-A- 3 710 789
- US-A- 4 905 679
- BIOMEDIZINISCHE TECHNIK, Band 31, Nr. 12, Dezember 1986, Seiten 303-307, Berlin, DE; Th. HEINL et al.: "Vorschlag für die analytisch begründete Konstruktion einer Osteosynthese-Miniplatte aus Titan"

## Beschreibung

Die Erfindung betrifft ein Gitter für die Osteosynthese oder zum Befestigen von künstlichen Körperteilen mit den Merkmalen des Oberbegriffs des Patentanspruchs 1.

Unter der Osteosynthese versteht man die operative Verbindung der Endstellen eines Knochenbruchs durch mechanische Hilfsmittel.

Bei Frakturen (Brüchen) und Osteotomien (operative Durchtrennung eines Knochens) insbesondere im Bereich des menschlichen Schädels ist eine sogenannte Fixation notwendig, d.h. eine feste Positionierung der Fragmente zueinander.

Zum Fixieren der miteinander zu verbindenden Knochenstücke sind im Stand der Technik verschiedene Plattensysteme und auch Drahtnähte bekannt.

Die Verwendung von Draht hat den Nachteil einer mangelhaften Funktionsstabilität. Drahtnähte kommen deshalb nur noch selten und bei kleinen Fragmenten zur Anwendung, wo jedoch die Einfädelung äußerst schwierig ist und oftmals zu einer völligen Denudierung des Knochens führt, d.h. einer unerwünschten Entfernung der Knochenhaut.

Bekannte Plattensysteme für die Osteosynthese haben gegenüber der Drahtnaht zwar Verbesserungen gebracht, sind jedoch ebenfalls mit Nachteilen behaftet. So muß die Fraktur relativ umfangreich freigelegt und präpariert werden, was den Heilungsprozeß verzögert. Auch haben bekannte Plattensysteme den Nachteil, daß eine Stabilisierung der Knochenfragmente im wesentlichen nur zweidimensional erfolgt. Werden aber mehrere großflächige Platten verwendet, so werden auch die über den Platten zum Liegen kornenden Weichteile vom Knochen abgehalten, was den Heilungsprozeß ebenfalls behindern kann. Überdies haben die bekannten Plattensysteme auch den Nachteil, daß sie nur mit sehr viel Aufwand exakt an den Knochenbau angepaßt werden können. Gelingt diese Anpassung nicht exakt, so besteht die Gefahr eines Verziehens der Knochenteile.

Aus dem DE-GM 87 06 912 sind sogenannte Kleinknochenplatten bekannt, bei denen zwischen einzelnen ringförmigen Lochfassungen Stege vorgesehen sind. Diese Kleinknochenplatten sind aber nicht netzförmig und sie decken im Verhältnis zu ihrer Gesamtgröße eine relativ große Knochenfläche ab. Entsprechendes gilt für die US-PS 2 494 229 und das DE-GM 85 28 003. Auch die EP 0 291 632 A1 beschreibt Kleinknochenplatten, bei der im Verhältnis zur Gesamtfläche der Knochenplatte ein relativ großer Anteil des Knochens abgedeckt wird.

Das DE-U-88 07 909 beschreibt eine Knochenplatte für die Osteosynthese mit Gittern zum Verankern der Platte, bei denen langgestreckte Stege zwischen einzelnen Schraublochfassungen vorgesehen sind. Dabei greifen aber freistehende Stege wie Arme nach außen und zwischen außenliegenden Schraubenlöchern sind keine Stege vorgesehen.

Bevor das vorstehend beschriebene Osteosynthesematerial mittels Schrauben od. dergl. am Knochen befestigt wird, ist die betroffene Knochenfläche freizulegen. In Folge dieser Denudierung des Knochens wird die Gefäßversorgung der betroffenen Knochenteile unterbrochen, was zu einem flächigen Abbau von Knochenmaterial im denudierten Bereich führen kann bis die Gefäßversorgung aufgrund des natürlichen Heilungsprozesses wieder hergestellt ist. Der Stand der Technik ist dahingehend verbesserungfähig, als dem Chirurgen die Möglichkeit gegeben werden sollte, die Knochenoberfläche nur so gering als möglich freizulegen. Und das Osteosynthesematerial sollte in montiertem Zustand nur eine relativ geringe Knochenfläche abdecken, damit während des Heilungsvorganges die Gefäßversorgung so schnell wie möglich und so flächendeckend wie möglich hergestellt werden kann. Ein Abbau von Knochen unter dem Osteosynthesematerial sollte soweit als möglich vermieden werden.

Neben der anzustrebenden möglichst geringen Flächenabdeckung des Osteosynthesematerials in bezug auf die Gesamtfläche des Gitters ist aber auch eine hohe mechanische Stabilität des Gesamtsystems aus im Knochen verschraubtem Gitter und dem zu verheilenden Knochen erforderlich. Die Forderungen nach möglichst geringer Flächenabdeckung einerseits und hoher mechanischer Stabilität andererseits sind gegenläufig.

Der vorliegenden Erfindung liegt die Aufgabe zugrunde, ein Gitter für die Osteosynthese sowie zum Befestigen von künstlichen Körperteilen, wie Epithesen, zu schaffen, welches einerseits einen günstigen Verlauf der Knochenheilung und andererseits eine hohe dreidimensionale Stabilität des Systems aus Gitter, Knochen und Schrauben ermöglicht.

Erfindungsgemäß wird diese Aufgabe dadurch gelöst, daß die Verbindungslinie der Mittelpunkte jeweils zweier beliebiger Löcher des Gitters innerhalb der äußeren Umrandung des Gitters liegt und daß das Gitter nicht mehr als 45 % des von ihm belegten Knochens abdeckt.

Bevorzugt ist das erfindungsgemäße Gitter so ausgestaltet, daß die Verbindungslinie von jeweils zwei beliebigen benachbarten Löchern, die am Rand des Gitters liegen, jeweils die Mittellinie eines Steges ist.

Die Erfindung beruht auf der Erkenntnis, daß der Chirurg dann optimale Ergebnisse hinsichtlich der Stabilität des Systems und gleichzeitig hinsichtlich des Heilungsverlaufes erzielen kann, wenn einerseits zur Förderung der dreidimensionalen Stabilität des Gitters die Verbindungslinie von allen möglichen Loch-Paaren des Gitters jeweils innerhalb der äußeren Umrandung des Gitters liegt und andererseits die Knochenabdeckung möglichst gering ist.

Das erfindungsgemäße Gitter kann vom Chirurgen vor Ort präpariert werden, um es an die Erfordernisses des Einzelfalles anzupassen. Dabei kann der Chirurg das Gitter so zurechtschneiden, daß zwischen zwei Randlöchern ein Steg stehen bleibt und so ein zweidimensionales Gitterstück verbleibt, von dem gegebenenfalls ausgreifende Arme abstehen. Wird dieses zweidimensionale, an seinen Rändern durch Stege stabilisierte Gebilde mit dem Knochen verschraubt, so entsteht insgesamt eine dreidimensionale stabile Struktur.

Weitere bevorzugte Ausgestaltungen des Erfindungsgegenstandes sind in den Unteransprüchen beschrieben.

Die Erfindung bezieht sich somit auf ein handelbares Produkt, welches so gestaltet ist, daß es durch einfache Manipulationen vor Ort vielfältig entsprechend den Anforderungen des Einzelfalles abwandelbar ist.

Mit durch die Löcher in den Knochen eingedrehten Schrauben entsteht als Grundgerüst ein Quader (dreidimensional).

Im Unterschied zum Stand der Technik, bei dem großflächige Platten mit einer Vielzahl von Löchern versehen sind, sieht also die Erfindung eine grazile Stegkonstruktion vor, wobei die Versteifung und die Stabilität durch schmale Stege erreicht wird. Eine solche gitterförmige Struktur läßt sich leicht an den individuellen Knochenbau anpassen und liefert trotzdem eine gute dreidimensionale Stabilität, insbesondere wenn Schrauben an den Ecken eines durch das Gitter gebildeten Vierecks durch die Löcher in den Knochen eingeschraubt werden.

Gemäß einer bevorzugten Ausgestaltung der Erfindung weisen die feinen Stege zumindest teilweise jeweils eine Länge von mehr als 4 mm und eine Breite von weniger als 1,5 mm, vorzugsweise weniger als 1 mm auf.

Gemäß einer anderen bevorzugten Weiterbildung der Erfindung ist vorgesehen, daß die Stege zumindest teilweise eine Länge von mehr als 5 mm aufweisen, wobei sie über diese gesamte Länge die vorstehend angegebenen Breiten aufweisen.

Eine besonders umfangreiche Gestaltungsfreiheit wird dem Chirurgen dann in die Hand gegeben, wenn die erfindungsgemäßen Gitter mit unterschiedlichen Steg-Strukturen, d.h. Längen der Stege und ihren geometrischen Anordnungen, bereitgestellt werden, so daß vor Ort die Gitter wahlweise durch Entfernen bestimmter Partien dem gegebenen Problem optimal angepaßt werden können.

Das erfindungsgemäße Gitter für die Osteosynthese hat gegenüber herkömmlichen Plattensystemen den Vorteil, daß die Stabilität der Fixation im wesentlichen durch die Form des Gitters bedingt ist, nicht aber durch die Stärke oder Länge der Platte. Das erfindungsgemäße Gitter kann so gestaltet werden, daß es einfach an die geforderte Geometrie der Osteosynthese anpaßbar ist. Es ist gut biegsam und wird erst nach der Verschraubung aufgrund der dreidimensionalen Versteifung stabil. Eine intermaxilläre Fixation ist nicht erforderlich.

Weiter hat das erfindungsgemäße Gitter den Vorteil, daß eine übermäßige Denudierung (Freilegung) des Knochens vermieden werden kann. Bei der Anbringung des erfindungsgemäßen Gitters braucht nur relativ wenig Periost abgeschoben zu werden. Da das Periost (bindegewebige Hautschicht um den Knochen) durch Nährstoffzufuhr zum Knochen, die das ganze Leben fortdauernde Knochenerneuerung (endostale Ossifikation) gewährleistet, kann bei einer erfindungsgemäßen Osteosynthese der Heilungsprozeß rascher und störungsunanfälliger erfolgen.

Da ein erfindungsgemäßes Gitter parallel zur Fraktur gelegt werden kann, muß in unmittelbarer Nähe des Fraktur- bzw. des Osteotomiespaltes das Periost abgeschoben werden. Auch dieser Umstand ermöglicht bei Verwendung eines erfindungsgemäßen Gitters eine bessere Blutversorgung der Fragmente und damit einen besseren Heilungsverlauf.

Weiter ermöglicht ein erfindungsgemäßes Gitter eine individuelle Anpassung an extrem komplizierte geometrische Anforderungen aufgrund des Bruches. Wird das erfindungsgemäße Gitter beispielsweise in Stücken von 6 x 6 cm Kantenlänge bereitgestellt, so kann der Chirurg vor Ort aus einem solchen Gitterstück je nach der vorliegenden Osteotomie bzw. dem Frakturverlauf gewünschte Stücke herausschneiden. Besonders bei Trümmerbrüchen sind auf diese Weise Kleinstfragmente mit einzelnen, individuell angebogenen Fingerfortsätzen gut fixierbar. Die Fragmente brauchen nicht durch zusätzliche Drahtnähte fixiert zu werden und auch eine Denudierung kann weitgehend vermieden werden.

Weiter haben erfindungsgemäße Gitter auch den Vorteil, daß bei ihrer Anbringung nicht große Areale von Gewebe freigelegt werden müssen. Deshalb ist auch die spätere Entfernung eines erfindungsgemäßen Gitters von der Bruchstelle relativ einfach und kann sogar in Lokalanästhesie durchgeführt verden. Auch beim Entfernen des Gitters muß nur das ehemalige Operationsgebiet geringfügig freigelegt werden.

Darüberhinaus hat das erfindungsgemäße grazile Gitter den Vorteil, nicht aufzutragen. Dies ist sowohl medizinisch als auch ästhetisch von Vorteil.

Eine Variante der Erfindung betrifft statt der Osteosynthese die Befestigung von Epithesen, insbesondere im Bereich des menschlichen Gesichtes. Das in den Ansprüchen beschriebene erfindungsgemäße Gitter ist auch für diesen Zweck geeignet.

Zur Befestigung von Epithesen (künstlichen Ohren, Nasen, Augen etc.) im Bereich des Gesichtes werden gemaß dem Stand der Technik in der Regel Hilfsmittel wie eine Brille oder eine Prothese verwendet.

Eine andere Lösung zum Befestigen von Epithesen im Bereich des Gesichtes sieht vor, daß einzelne Implantate im Schädelknochen fixiert werden, an denen dann die Epithese befestigt wird, wie bei Implantaten in der Mundhöhle zur Fixation von Zahnprothesen. Der Nachteil eines solchen Implantatsystems liegt vor allem darin, daß genügend Knochen vorhanden sein muß, um die Implantatpfosten aufnehmen zu können. So ist es häufig nicht möglich, am Ort der zu befestigenden Epithese die Implantatpfosten zu setzen. Auch haben solche Implantatpfosten den Nachteil, daß sie ein sogenanntes "zweistufiges" Verfahren erfordern, wobei zunächst der Implantatpfosten in den Knochen eingefügt wird und dort eine längere Zeit ohne Belastung durch die Epithese verbleibt, um einheilen zu können. Erst danach wird mit zeitlichem Abstand von einigen Monaten dann auf dem oder den Implantatpfosten die Epithese bei einer zweiten Operation befestigt.

Auch haben bekannte Vorrichtungen zum Befestigen von Epithesen den Nachteil, daß die Stellung und die Form der Epithese nicht immer optimal sein kann, da man beim Setzen der Implantate ungünstigen Randbedingungen unterliegt, d.h. die Implantate nicht dort setzen kann, wo sie für einen idealen Sitz der Epithese wünschenswert wären.

Ein solches erfindungsgemäßes Gitter zum Befestigen von Epithesen weist alle oben in Zusammenhang mit der Osteosynthese genannten Vorteile auf.

Darüberhinaus hat das erfindungsgemäße Gitter beim Befestigen von Epithesen den Vorteil, daß mit zwei bis drei kurzen Schrauben das Gitter am festen Knochen verankert werden kann. Durch die Verwendung von kurzen Schrauben sind keine dicken Knochenstrukturen zum Befestigen der Epithese notwendig und es werden auch Störungen der Infrastruktur des Knochens weitgehend vermieden. Dies ist insbesondere im Bereich des Gehirnschädels und bei Kindern wichtig.

Das erfindungsgemäße Gitter wird mit relativ kurzen 2- bis 4-mm-Schrauben am Knochen befestigt, wobei eine sehr gute Stabilität gegeben ist, da die dünnen Gitter-Stege zwischen den Schrauben sehr schnell vom Knochen überwachsen werden.

Ein weiterer wesentlicher Vorteil des erfindungsgemäßen Gitters liegt darin, daß es an einem anderen Ort am Knochen befestigbar ist als der für die Epithese vorgesehene Ort. Ein erfindungsgemäßes Gitter kann wie ein "Kran" derart eingesetzt werden, daß es mit bestimmten Teilen an einem günstigen Knochenstück befestigt wird, während z.B. einige Zentimeter davon entfernt dann an einem Ausleger des Gitters die Epithese befestigt werden kann. Auf der Basis dieses "Kran"- bzw."Auslegerprinzips" ist es also möglich, eine Epithese an einem Ort zu positionieren und mit Hilfe des erfindungsgemäßen Gitters zu befestigen, an dem unterhalb der Epithese gar kein Knochen zur Verfügung steht.

Auch ermöglicht es das erfindungsgemäße Gitter, die Epithesenstellung individuell anzupassen.

Weiterhin kann mit einem erfindungsgemäßen Gitter die Epithese in einem einzigen Arbeitsgang installiert werden, da die Befestigung des Gitters am Knochen an ganz anderer Stelle stattfinden kann als am Ort der zu fixierenden Epithese, d.h. die Durchtrittsstelle durch die Haut ist nicht durch den Ort der Befestigung der Epithese festgelegt, sondern kann an eine günstigere Stelle verlegt werden.

Alle diese Möglichkeiten standen bei herkömmlichen Schraubenimplantaten nicht zur Verfügung.

Nachfolgend wird die Erfindung anhand der Zeichnung näher erläutert. Es zeigt:
- Fig. 1: eine Draufsicht auf ein Gitter;
- Fig. 2: eine Draufsicht auf ein anderes Ausführungsbeispiels eines Gitters;
- Fig. 3: ein weiteres Ausführungsbeispiel eines Gitters mit abgewandelten Stegformen; und
- Fig. 4, 5 und 6: Gitter gemäß Fig. 1, die vom Benutzer zurechtgeschnitten sind.

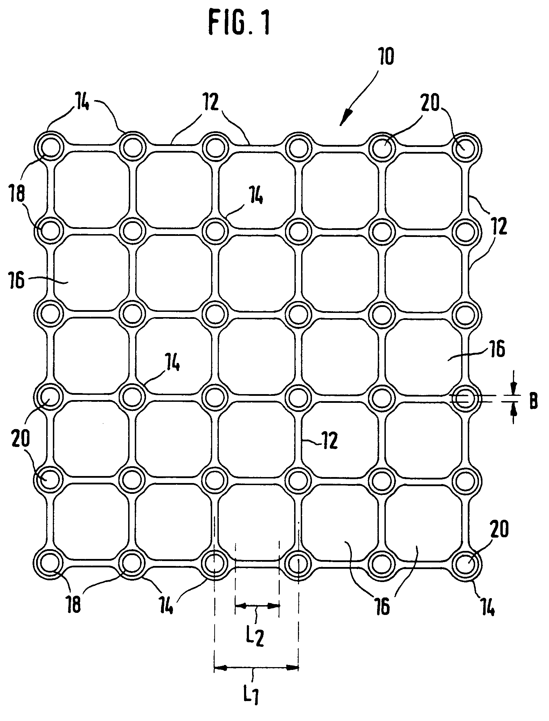

Das in Fig. 1 gezeigte Gitter 10 ist gegenüber seinem realen Maßstab zweifach vergrößert, d.h. die reale Kantenlänge beträgt etwa 5 cm x 5 cm.

Das Gitter 10 weist eine Vielzahl von jeweils einzelne Quadrate bildenden Stegen 12 auf. Das Gitter besteht insgesamt einstückig aus Titan.

Die einzelnen Stege 12 sind jeweils matrixförmig über Ringe 14 miteinander verbunden. Die Ringe 14 sind gerade so groß, daß sie Löcher 20 aufnehmen können, durch welche z.B. Schrauben durchsteckbar sind. Die Löcher 20 können auch mit Innengewinde versehen sein, um die Befestigung von Epithesen oder dergleichen zu ermöglichen.

Zwischen den Stegen 12 sind rechteckförmige Freiräume 16 freigelassen.

Die Löcher 20 sind jeweils mit Ansenkungen 18 versehen, so daß Schrauben koplanar mit der Oberfläche des Gitters versenkt werden können.

Der Abstand L₁ zweier benachbarter Steg-Kreuzungspunkte beträgt beim in Fig. 1 dargestellten Ausführungsbeispiel 1 cm. Abweichungen hiervon von ± 2 mm ermöglichen ebenfalls günstige Stegstrukturen. Die Stege 12 sind über ihre gesamte freie Länge L₂ relativ schmal, beim dargestellten Ausführungsbeispiel beträgt ihre Breite B nicht mehr als 1 mm.

Die Höhe (in der Zeichnungsebene) des gesamten Gitters ist gleichmäßig und beim dargestellten Ausführungsbeispiel nicht größer als 1 mm.

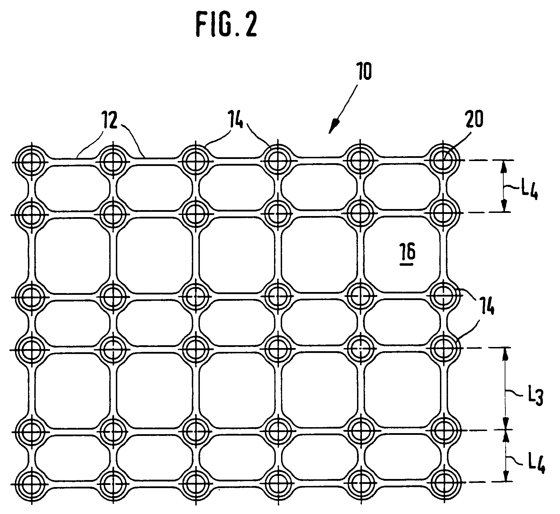

Fig. 2 zeigt eine Abwandlung des in Fig. 1 dargestellten Gitters, wobei die Länge der Stege unterschiedlich ist, was dem Chirurgen erweiterte Gestaltungsfreiheit beim Einsatz des Gitters ermöglicht. Das in den Fig. 1 und 2 dargestellte Gitter kann vor Ort mit einfachen Werkzeugen gemäß den Anforderungen zurechtgeschnitten und -gebogen werden (siehe oben).

Die unterschiedlichen Längen L₃ und L₄ gemäß Fig. 2 ermöglichen einerseits eine Konzentration von Schrauben bei beengten Verhältnissen und andererseits die Überbrückung längerer Strecken mit wenig Material.

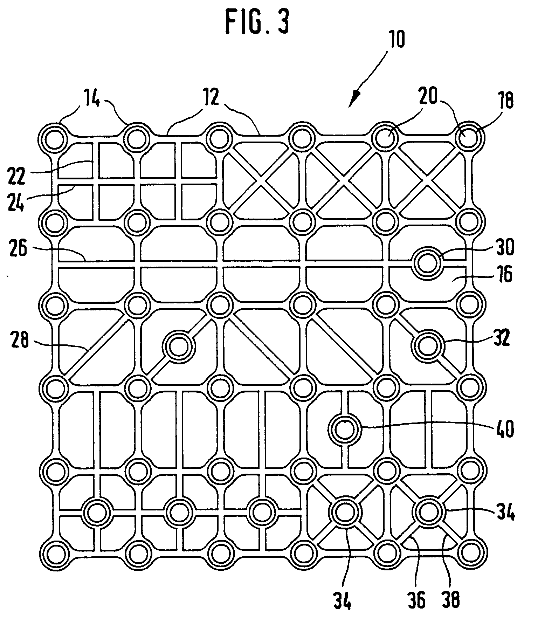

Fig. 3 zeigt Abwandlungen der Steg- und Ringstruktur. Das Gitter 10 weist sehr unterschiedliche Anordnungen von Ringen und Stegen auf, wobei die Stege 22, 24 nicht zwischen Ringen 14 verlaufen, sondern eine zusätzliche Stabilisierung des Gitters in sich bewirken sollen. Der Aufbau des in Fig. 3 gezeigten Gitters versteht sich von selbst ohne Beschreibung. Die dargestellte Struktur ist Teil der Erfindung.

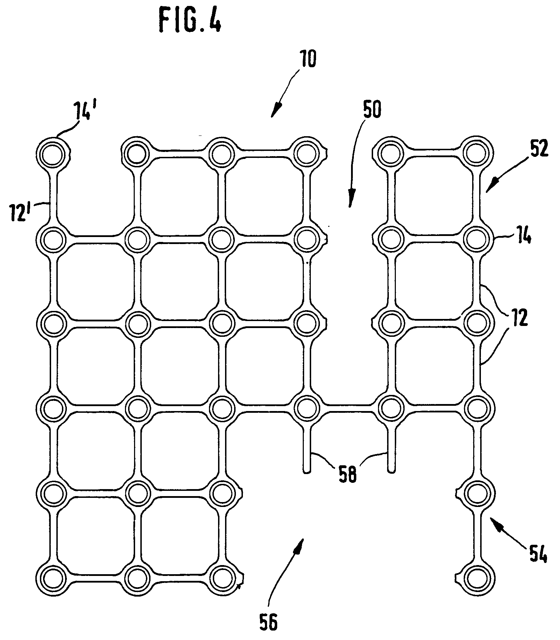

Fig. 4 zeigt ein Gitter gemäß Fig. 1, das beispielhaft zurechtgeschnitten ist, um die unterschiedlichen Einsatzmöglichkeiten eines Grundgitters gemäß den Fig. 1 bis 3 zu veranschaulichen.

In das Gitter 10 gemäß Fig. 4 ist zum Beispiel eine Lücke 50 durch Entfernen der Stege 12 geschnitten. Hierdurch entsteht ein freier Arm 52, der vom Chirurgen leicht an die Erfordernisse geometrisch anpaßbar ist. Durch Wegnahme von Stegen 12 und Ringen 14 kann gemäß Fig. 4, rechts unten, ein freier Finger 54 geschaffen werden, der wie ein Ausleger von einem vier Löcher zum Befestigen von Schrauben aufweisenden Grundstück absteht. In eine Lücke 56 ragen freie Stege 58, die kleinere Fragmente oder Trümmer halten können. Weiterhin zeigt Fig. 4, links oben, einen freistehenden Ring 14' an einem Steg 12'.

Durch die Wegnahme von Stegen und die Bildung der in Fig. 4 gezeigten Lücken ist es möglich, die Denudierung des Knochens auf das unumgängliche Maß zu beschränken.

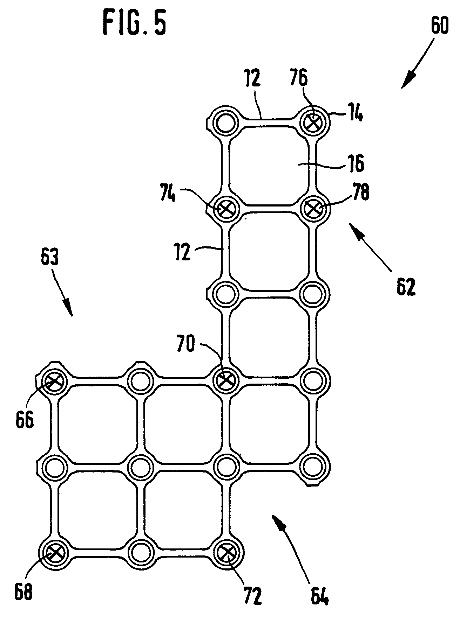

Fig. 5 zeigt ein Gitter 60, das ausgehend von einem Gitter gemäß Fig. 1 präpariert worden ist. Das Gitter 60 ist so gestaltet, daß es zum einen einen Ausleger 62 aufweist, an dem später die Epithese befestigt werden soll, sowie ein Grundteil 63, das mit Schrauben 66, 68, 70, 72 am Knochen befestigt ist. Dort wo keine Befestigung erforderlich ist, ist eine Lücke 64 gelassen.

Schrauben 74, 76, 78 dienen zur Befestigung einer Epithese am Gitter 60. Fig. 5 zeigt, daß der Ort der Befestigung des Gitters (Grundteil 63) recht weit vom Ort der Befestigung der Epithese (Ausleger 62) entfernt sein kann. Ausgehend von einem Gitter gemäß einer der Fig. 1 bis 3 sind vielfältige Gestaltungsmöglichkeiten gegeben.

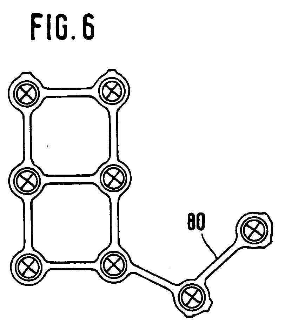

Fig. 6 zeigt eine Konfiguration, die besonders bei Trümmerbrüchen mit Kleinstfragnenten vorteilhaft ist. Mit einzelnen, individuell angebogenen Fingerfortsätzen 80 gemäß Fig. 6 können solche Kleinstfragmente gut fixiert werden. Der Fingerfortsatz (Verlängerungsarm) 80 kann vom Chirurgen wunschgemäß abgewickelt werden. Solche individuell angebogenen Fingerfortsätze ermöglichen in allen drei Ebenen eine Anpassung an die Anatomie des Knochens.

Die vorstehend beschriebenen Gitterkonfigurationen zeichnen sich gegenüber dem Stand der Technik zum einen dadurch aus, daß sie konvex sind und zum anderen dadurch, daS sie eine relativ geringe Flächenabdeckung bezüglich des Knochens bewirken. Der Begriff "konvex" bedeutet, daß das Gitter in seiner gehandelten Form so gestaltet ist, daß die Verbindungslinie der Mittelpunkte zweiter beliebiger Loch-Paare innerhalb der äußeren Umrandung des Gitters liegt, d.h. innerhalb der äußeren Kontur der materiellen Teile des Gitters. Dabei soll die Verbindungslinie von jeweils zwei beliebigen benachbarten Rand-Löchern des Gitters möglichst mit einem Steg zusammenfallen, wodurch die dreidimensionale Stabilität eines gegebenfalls zurechtgeschnittenen Gitters, das mit einem zu verheilenden Knochen verschraubt ist, erheblich gefördert wird. Gleichzeitig beträgt die Flächenabdeckung des Gitters bezüglich des Knochens nicht mehr als 45 %, bevorzugt nicht mehr als 35 % und noch weiter bevorzugt nicht mehr als 30 %. Der Begriff "Flächenabdeckung" beschreibt in % das Verhältnis der Gesamtfläche zwischen den äußeren Konturen des Gitters einschließlich der Perforationen zur Gesamtfläche des Metalles. Dabei können die Flächen durch Parallel-Projektion auf eine Ebene oebildet werden, die parallel zur Hauptebene des Gitters liegt, d.h. in die Berechnung des Abdeckungsgrades des Gitters gehen nur zweidimendionale Erstreckungen des Gitters in seiner Hauptebene ein, nicht aber Profilierungen des Gitters senkrecht zur Hauptebene.

## Patentansprüche

1. Gitter für die Osteosynthese oder zum Befestigen von künstlichen Körperteilen, wie Epithesen, mit einer Mehrzahl von netzartig angeordneten Stegen (12), wobei an zumindest einem Teil der Kreuzungspunkte der Stege Löcher (20) für Befestigungsschrauben vorgesehen sind,
dadurch **gekennzeichnet,** daß
die gerade Verbindungslinie von jeweils zwei beliebigen benachbarten Löchern (20), die am Rand des Gitters (10) liegen, jeweils die Mittellinie eines Steges (12) ist und innerhalb der äußeren Umrandung des Gitters liegt, und daß das Gitter einen Knochen-Abdeckungsgrad von nicht mehr als 45% aufweist.

2. Gitter nach Anspruch 1,
dadurch **gekennzeichnet,** daß
zumindest ein Teil der Stege (12) jeweils eine Länge (L₂) von mehr als 4 mm und eine Breite (B) von weniger als 1,5 mm aufweist.

3. Gitter nach Anspruch 1,
dadurch **gekennzeichnet,** daß
zumindest ein Teil der Stege (12) jeweils eine Länge (L₂) von mehr als 4 mm und eine Breite (B) von weniger als 1,5 mm aufweist.

4. Gitter nach einem der vorhergehenden Ansprüche,
dadurch **gekennzeichnet,** daß
die Stege (12) eine Höhe kleiner als 1,2 mm, vorzugsweise kleiner als 1 mm aufweisen.

5. Gitter nach einem der vorhergehenden Ansprüche,
dadurch **gekennzeichnet,** daß
zumindest ein Teil der Löcher (20) ein Innengewinde aufweist.

6. Gitter nach einem der vorhergehenden Ansprüche,
dadurch **gekennzeichnet,** daß
das Gitter im Bereich zumindest eines Teils der Kreuzungspunkte der Stege (12) Ringe (14) zur Aufnahme der Löcher (20) aufweist.

7. Gitter nach einm der vorhergehenden Ansprüche,
dadurch **gekennzeichnet,** daß
seine äußere Umrandung quadratisch, rechteckförmig, kreisförmig, dreieckförmig, ellipsenförmig oder rautenförmig ist.

8. Gitter nach einem der vorhergehenden Ansprüche,
dadurch **gekennzeichnet,** daß
es nicht mehr als 35%, vorzugsweise nicht mehr als 30% des von ihm belegten Knochens abdeckt.

## Claims

1. A grid for osteosynthesis or for fastening artificial body parts, such a epitheses, comprising a plurality of webs (12) arranged in a net pattern, with holes (20) for fastening screws provided at at least some of the intersections of the webs, **characterized** in that the straight connecting line of any two adjacent holes (20) located at the edge of the grid (10) is the respective center line of a web (12) and lies within the external boundary of the grid, and in that the grid has a bone covering degree of no more than 45 %.

2. The grid as claimed in claim 1, characterized in that at least some of the webs (12) each have a length (L₂) of more than 4 mm and a width (B) of less than 1.5 mm.

3. The grid as claimed in claim 1, characterized in that at least some of the webs (12) each have a length (L₂) of more than 4 mm and a width (B) of less than 1.5 mm.

4. The grid as claimed in any one of the preceding claims, characterized in that the webs (12) have a height of less than 1.2 mm, preferably less than 1 mm.

5. The grid as claimed in any one of the preceding claims, characterized in that at least some of the holes (20) have an internal thread.

6. The grid as claimed in any one of the preceding claims, characterized in that the grid comprises rings (14) to take up the holes (20) in the region of at least some of the intersections of the webs (12).

7. The grid as claimed in any one of the preceding claims, characterized in that its external boundary is square, rectangular, circular, triangular, elliptical, or rhombic.

8. The grid as claimed in any one of the preceding claims, characterized in that it covers no more than 35 %, preferably no more than 30 % of the bone it occupies.

## Revendications

1. Grille destinée à l'ostéosynthèse ou à la fixation de prothèses corporelles telles qu'éléments correcteurs de difformités, comportant une multiplicité de traverses (12) disposées en réseau, des trous (20) pour des vis de fixation étant prévus à au moins une partie des points d'intersection des traverses, caractérisée en ce que la droite reliant deux trous voisins quelconques (20) situés sur le bord de la grille (10) est l'axe médian d'une traverse (12) et est située à l'intérieur de la bordure externe de la grille et en ce que la grille présente un degré de couverture de l'os inférieur ou égal à 45 %.

2. Grille selon la revendication 1, caractérisée en ce qu'au moins une partie des traverses (12) a une longueur (L₂) supérieure à 4 mm et une largeur (B) inférieure à 1,5 mm.

3. Grille selon la revendication 1, caractérisée en ce qu'au moins une partie des traverses (12) a une longueur (L₂) supérieure à 4 mm et une largeur (B) inférieure à 1,5 mm.

4. Grille selon une des revendications précédentes, caractérisée en ce que les traverses (12) ont moins de 1,2 mm de haut, de préférence moins de 1 mm.

5. Grille selon une des revendications précédentes, caractérisée en ce qu'au moins une partie des trous (20) présente un taraudage.

6. Grille selon une des revendications précédentes, caractérisée en ce que la grille présente, dans la zone d'au moins une partie des points d'intersection des traverses (12), des anneaux (14) destinés à loger les trous (20).

7. Grille selon une des revendications précédentes, caractérisée en ce que sa bordure externe est carrée, rectangulaire, circulaire, triangulaire, ellipsoïdale ou en losange.

8. Grille selon une des revendications précédentes, caractérisée en ce qu'elle couvre un pourcentage inférieur ou égal à 35 %, de préférence à 30 %, de l'os qu'elle occupe.
